# EUROPÄISCHE PATENTSCHRIFT

(11) **EP 2 452 606 B1**
(45) Veröffentlichungstag und Bekanntmachung des Hinweises auf die Patenterteilung: **02.09.2015**
(21) Anmeldenummer: 11008984.4
(22) Anmeldetag: 11.11.2011
(51) Int. Cl.: A47L 15/23, A61B 19/00

(54) **Rotations-Überwachung für die Wascharme eines mehretagigen Waschgut-Aufnahmewagens**
Rotation monitoring for the washing arms of a multi-level laundry vehicle
Surveillance de rotation pour les bras de lavage d'un chariot de lavage à plusieurs etages

(30) Priorität: 16.11.2010 DE 102010051524
(43) Veröffentlichungstag der Anmeldung: 16.05.2012
(73) Patentinhaber: Belimed AG, 6300 Zug (CH)
(72) Erfinder: Rieder, Roman, 7205 Zizers (CH); Küttel, Philipp, 6362 Stansstad (DE)
(74) Vertreter: Feldkamp, Rainer

(56) Entgegenhaltungen:
- EP-A1- 1 774 979
- EP-A2- 1 743 566
- WO-A1-2011/154813
- DE-B3-102005 059 604

## Beschreibung

Die Erfindung bezieht sich auf eine Reinigungsmaschine zum Reinigen von medizinischen, pharmazeutischen und/oder Labor-Utensilien der im Oberbegriff des Anspruchs 1 genannten Art.

Es sind Reinigungsmaschinen dieser Art bekannt, bei denen ein Waschgut-Aufnahmewagen in den Waschraum der Reinigungsmaschine, beispielsweise nach Art einer Geschirrspülmaschine oder einer Maschine zum Reinigen von medizinischen, pharmazeutischen und/oder Labor-Utensilien einsetzbar ist, wobei der Begriff "Reinigung" hierbei sowohl das Beaufschlagen der einzelnen Utensilien oder Gegenstände mit einer , Waschflüssigkeit, einer Spülflüssigkeit und gegebenenfalls Trocknungsluft einschließen soll. Derartige Waschgut-Aufnahmewagen weisen zumeist mehrere übereinander angeordnete Etagen oder Ebenen auf, in denen das Waschgut entweder direkt oder in körben angeordnet werden kann, wobei jeder dieser Ebenen zumindest ein Wascharm zugeordnet ist, der um eine vertikale Drehachse drehbar ist, die gleichzeitig zur Zuführung eines Strömungsmediums, wie der Waschflüssigkeit oder Trocknungsluft, über Kanäle dient, die beim Einschieben des Waschgut-Aufnahmewagens in den Waschraum mit einer in dem Waschraum angeordneten Zuführungs- und Verteilereinrichtung verbunden werden.

Durch entsprechende Gestaltung der Austrittsrichtung der Strömungsmedien-Strahlen aus den Wascharmen wird ein Antrieb der Wascharme um deren Drehachse erreicht.

Die Drehung der einzelnen Wascharme kann jedoch durch das auf den einzelnen Ebenen befindliche Waschgut bei fehlerhafter Anordnung hiervon beeinträchtigt werden, wodurch die Reinigungswirkung stark beeinträchtigt wird.

Aus diesem Grunde ist es üblich, Überwachungseinrichtungen für die Drehung der Wascharme vorzusehen, die ein Stoppen der Maschine bei fehlender Drehung eines oder mehrerer der Wascharme ermöglichen.

Aus der EP 1743566 A2 ist ein Überwachungssystem bekannt, bei dem an den Enden der Wascharme angeordnete Magnete mit an einer Seitenwand des Waschraums angeordneten Magnetdetektoren zusammenwirken. Zur Erkennung, an welchen Höhenpositionen bei einem bestimmten Waschwagensignale von Sprüharmen zu erwarten sind, ist eine Eingabevorrichtung zur Eingabe einer Information vorgesehen, die die jeweiligen Höhenpositionen der Wascharme angibt.

Diese Überwachungseinrichtungen sind zum Teil relativ aufwändig und nicht für alle Anwendungsfälle geeignet.

Der Erfindung liegt die Aufgabe zugrunde, eine Rotations-Überwachung für Wascharme eines Waschgut-Aufnahmewagens einer Reinigungsmaschine der eingangs genannten Art zu schaffen, die bei einfachem Aufbau zu erkennen lässt, in welcher Ausrichtung der Aufnahmewagen in den Waschraum eingeschoben wurde.

Diese Aufgabe wird durch die im Patentanspruch 1 angegebenen Merkmale gelöst.

Vorteilhafte Ausgestaltungen und Weiterbildungen der Erfindung ergeben sich aus den Unteransprüchen.

Bei der erfindungsgemäßen Rotations-Überwachung der Reinigungsmaschine sind an den beiden Seitenwänden des Waschraums Sensorrohre angeordnet, die selbst bei einem Aufnahmewagen, bei dem die Drehachsen der Wascharme gegenüber einer in Einschubrichtung gesehenen Mittelebene des Waschraums versetzt sind, eine sichere Überwachung der Drehung der Wascharme ermöglichen. In Abhängigkeit von der seitlichen Versetzung der Drehachsen gegenüber der genannten Mittelebene werden die Detektoren des jeweils den Enden der Wascharme nächstgelegenen Sensorrohre betätigt, wobei dies weiterhin eine Erkennung einerseits der Ausrichtung und Art des Aufnahmewagens und andererseits der zuverlässigen Drehung der Wascharme ermöglicht.

Die Sensorrohre enthalten vorzugsweise Magnetdetektoren, beispielsweise in Form von Reed-Schaltern, wobei an zumindest einem freien Ende eines Wascharms ein Magnet angeordnet ist, der beim Vorbeilaufen an den in den Sensorrohren angeordneten Magnetdetektoren deren Betätigung bewirkt.

Gemäß einer bevorzugten Ausgestaltung der Erfindung sind die an den Enden der Wascharme angeordneten Magnete derart ausgebildet, dass lediglich die aufgrund des Versatzes zwischen den Drehachsen der Wascharme und der Mittelebene des Waschraums nächstliegenden Magnetdetektoren betätigt werden.

Die Magnete können in vorteilhafter Weise seitlich neben den Enden der Wascharme angeordnet sein, so dass im Bereich der Enden der Wascharme zusätzliche Düsen angeordnet werden können, die vorzugsweise in Endkappen der Wascharme ausgebildet sind, die das freie Ende der Wascharme verschließen. Diese Endkappen können vorzugsweise gleichzeitig zur Halterung der seitlich angeordneten Magnete verwendet werden.

Gemäß einer weiteren vorteilhaften Ausgestaltung der Erfindung sind jeder denkbaren Höhenlage der einzelnen Wascharme jeweils zwei vertikal übereinander in den Sensorrohren angeordnete Magnetdetektoren zugeordnet, die eine Vergrößerung des Überwachungsbereiches in vertikaler Richtung ermöglichen und damit eine sichere Überwachung selbst bei Toleranzen der Höhenlage der einzelnen Wascharme ergeben.

Die Erfindung wird im Folgenden anhand von in der Zeichnung dargestellten Ausführungsbeispielen noch näher erläutert.

In der Zeichnung zeigen:
Figur 1 eine perspektivische schematische Ansicht einer Ausführungsform der Reinigungsmaschine mit einem Waschraums mit einem darin eingeschobenen Waschwagen;
Figur 2 eine vereinfachte Vorderansicht des Waschraums und des Waschwagens nach Figur 1;
Figur 3a und 3b vereinfachte Darstellungen der Position des Aufnahmewagens gegenüber an den Seitenwänden des Waschraums angeordneten Sensorrohren in zwei um eine vertikale Achse um 360 Grad gedrehten Stellungen des Aufnahmewagens;

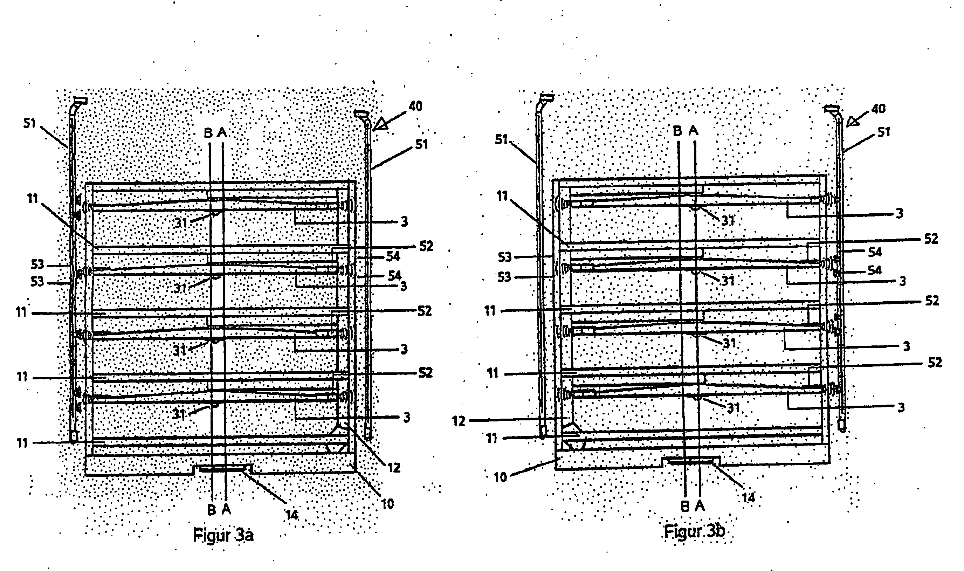

Figur 3 eine Ansicht eines Endes eines Wascharmes;

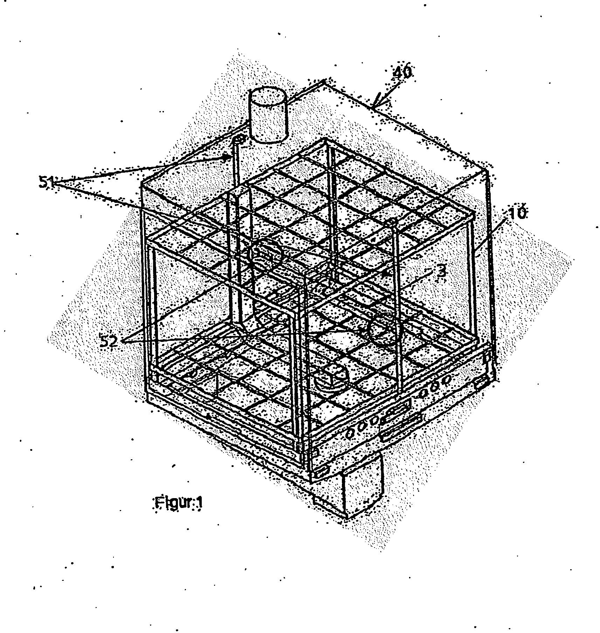

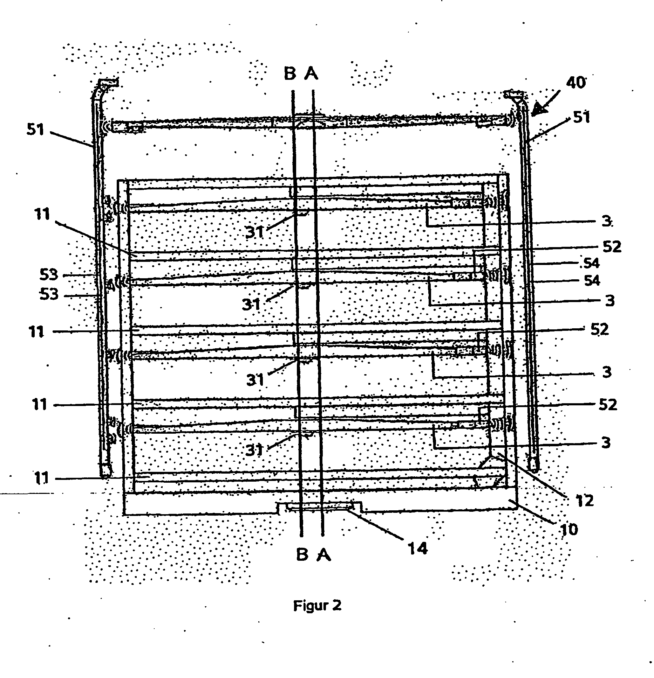

In Figur 1 ist schematisch ein Teil einer Reinigungsmaschine zum Reinigen von medizinischen, pharmazeutischen und/oder Labor-Utensilien gezeigt, die einen Waschraum 40 umfasst, in dem ein Aufnahmewagen 10 zur Aufnahme von medizinischen, pharmazeutischen und/oder Labor-Utensilien (1) entlang der Richtung E nach Figur 1 einschiebbar ist, wobei diese Utensilien auf in Figur 2 gezeigten Ebenen oder Etagen 11 des Aufnahmewagens 10 oder auf darauf gehalterten Aufnahmekörben anzuordnen sind.

Jeder Ebene oder Etage 11 des Aufnahmewagens 10 ist zumindest ein Wascharm 3 zugeordnet, der über jeweilige Drehkupplungen 31 mit Zufuhrkanälen 12 für Waschflüssigkeit, Spülflüssigkeit und/oder Trocknungsluft gespeist wird. Die Zuführung dieser Strömungsmedien erfolgt dabei über einen an einer Seite des Aufnahmewagens 10 angeordneten vertikalen Kanal 12, der über eine Kupplungseinrichtung 14 mit dem Waschraum 40 der Maschine verbunden ist.

Durch die Anordnung dieses vertikalen Kanals 12 auf einer Seite des Waschwagens 10 sind die Drehachsen der Wascharme 3 in der in den Figuren 2 und 3a, 3b gezeigten Weise entlang einer vertikalen Linie B ausgerichtet, die gegenüber der Mittelebene A sowohl des Aufnahmewagens 10 als auch des Waschraums (40) versetzt angeordnet ist.

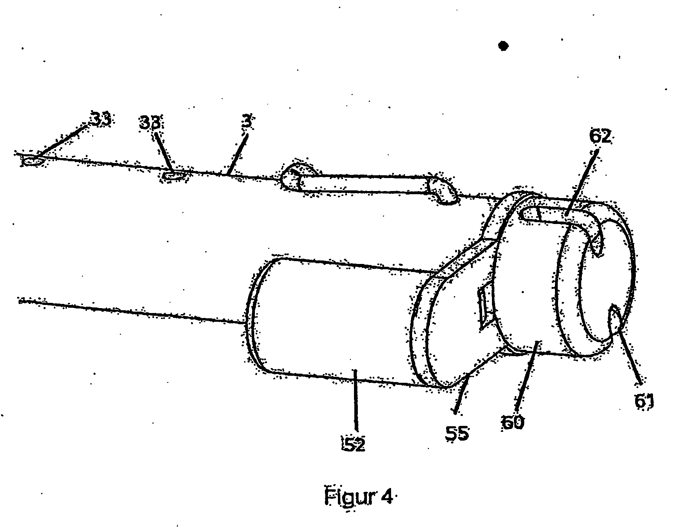

Um einen Einschub des Aufnahmewagens (10) in den Waschraum 40 in einer von zwei um 180° gegeneinander um eine vertikale Achse versetzten Positionen zu ermöglichen, von denen die erste Position in den Figuren 2 und 3b gezeigt ist, während die zweite Position lediglich in Figur 3a gezeigt ist, sind auf beiden Seitenwänden des Waschraums 40 Sensorrohre 51 angeordnet, die mit an den Enden der Wascharme 3 vorgesehenen Magneten 52 gemäß Figur 4 zusammenwirken.

Um weiterhin die Drehung der Wascharme (3) in unterschiedlichen Höhenlagen, je nach Aufnahmewagen, erfassen zu können, enthalten diese Sensorrohre 51 an jeder denkbaren Position der Wascharme vorzugsweise jeweils zwei Magnetdetektoren 53, 54 auf, die den Bereich sowohl unterhalb als auch oberhalb einer Drehebene eines denkbaren Wascharmes 3 erfassen.

Die Stärke der Magnete 52 eines jeweiligen Wascharms 3 und die Empfindlichkeit der einzelnen Magnetdetektoren 53 ist hierbei so gewählt, dass bei der in den Figuren 2 und 3a, 3b gezeigten Anordnung lediglich diejenigen Magnetdetektoren betätigt werden, die auf Grund des Versatzes zwischen der Linie B der Drehachsen und der Mittelebene A des Waschraums den Enden der Wascharme (3) am nächsten liegen.

Dies bedeutet, dass in der Position des Aufnahmewagens nach den Figuren 2 und 3b die Detektoren 53, die in dem Detektorrohr 51 angeordnet sind, betätigt werden. Hierbei weisen die Detektoren 54 in dem rechten Detektorrohr 51 gemäß Figur 2 einen größeren Abstand von den Enden der Wascharme (3) auf und werden durch diese nicht betätigt.

Bei der in Figur 3a gezeigten Ausrichtung des Aufnahmewagens werden entsprechen nur die Magnetdetektoren 54 betätigt, da diese dann den Enden der Wascharme am nächsten liegen.

Auf diese Weise ist es möglich, neben der Überwachung der einwandfreien Drehung der Wascharme einerseits die Höhenlage von in einem bestimmten Aufnahmewagen 10 vorhandenen Wascharmen 3 festzustellen, und andererseits festzustellen, in welcher Ausrichtung der Aufnahmewagen 10 in den Waschraum 40 eingeschoben wurde. Damit wird ein Einschieben des Aufnahmewagens 10 beliebig in zwei um 180 ° versetzten Ausrichtungen in den Waschraum 40 ermöglicht.

Die einzelnen Sensoren 53, 54 sind vorzugsweise als Reed-Schalter ausgerichtet, die jeweils beim Vorbeilaufen der Magnete 52 an den Enden der Wascharme 3 betätigt werden.

Um eine sichere Signalgabe der Magnetdetektoren selbst bei Toleranzen der Höhenlage der Drehebene der Wascharme 3 sicher zu stellen, sind vorzugsweise jeder denkbaren Höhenlage zwei über bzw. unter der Drehebene angeordnete Magnetdetektoren 53, 54 zugeordnet, wodurch der Erfassungsbereich vergrößert wird.

Durch die in Figur 4 gezeigte Anordnung der Magnete 52 an einem Halter 55 in einer gegenüber der Längsachse der Wascharme 3 seitlich versetzten Position können diese Wascharme mit Endkappen 60 versehen sein, die die Anordnung von Austrittsdüsen 61, 62 für zusätzliche Strahlen des Strömungsmediums ermöglichen, ohne dass diese Strahlen durch die Magnete 52 behindert werden.

Wenn in dem Waschraum 40 obere und untere , im Waschraum 40 selbst drehbar angeordnete Wascharme angeordnet sind, können die Sensorrohre 51 entsprechend nach oben und/oder nach unten hin verlängert und mit entsprechenden zusätzlichen Magnetdetektoren versehen sein, um auch die Drehung dieser Wascharme zu überwachen.

Wenn andererseits anstelle der jeder Ebene oder Etage 11 des Aufnahmewagens 10 zugeordneten, getrennt drehbaren Wascharme ein (nicht gezeigter) einteiliger Wascharm mit einem vertikalen Tragrohr und einer Anzahl von daran starr befestigten Wascharm-Hälften verwendet wird, kann es ausreichend sein, lediglich die Drehung eines Teils der Wacharm-Hälften zu überwachen.

## Patentansprüche

1. Reinigungsmaschine zum Reinigen von medizinischen, pharmazeutischen und/oder Labor-Utensilien, mit einem Waschraum (40) und einem Waschgut-Aufnahmewagen (10), welcher in den Waschraum (40) einsetzbar ist, wobei der Waschgut-Aufnahmewageneinzelne übereinander angeordnete Ebenen aufweist, auf welchen die Labor-Utensilien angeordnet werden können, wobei jeder Ebene jeweils ein Wascharm (3) zugeordnet ist, die Wascharme (3) um eine vertikale Drehachse (B) drehbar sind und über eine Drehkupplung mit Waschflüssigkeit gespeist werden können, wobei die Wascharme mit Austrittsöffnungen (33) versehen sind, aus denen Waschflüssigkeits-Strahlen auf die auf den einzelnen Ebenen (11) des Aufnahmewagens (10) befindlichen Gegenstände lenkbar sind, wobei die Reinigungsmaschine weiter ein Rotations-Überwachungssystem aufweist, welches an zumindest einer Seitenwand des Waschraums (40) angeordnete Magnetdetektoren aufweist, und wobei an den Enden der Wascharme Magnete angeordnet sind, die zur Betätigung der Magnetdetektoren (53, 54) beim Vorbeilaufen des Endes der Wascharme (3) an diesen ausgebildet sind,
**dadurch gekennzeichnet, dass** die Drehachsen (8) der Waschwarme des in dem Waschraum (40) eingeschobenen Aufnahmewagens (10) gegenüber der in Einschubrichtung des Waschwagens (10) verlaufenden Mittelebene (A) des Waschraums (40) versetzt sind, dass an beiden Seitenwänden des Waschraums (40) Sensorrohre (51) angeordnet sind, in denen eine Anzahl der einer möglichen Drehebene des Wascharms (3) zugeordneten Magnetdetektoren (53, 54) angeordnet sind, deren Erfassungsbereich die Drehebene eines jeweiligen Wascharms und den unmittelbar darüber und darunter liegenden Bereich erfasst, und dass die an den Enden der Wascharme (52) angeordneten Magnete (52) derart ausgebildet sind, dass lediglich die aufgrund des Versatzes zwischen den Drehachsen (B) der Wascharme (3) und der Mittelebene (A) des Waschraums (40) nächstliegenden Magnetdetektoren betätigt werden.

2. Reinigungsmaschine nach Anspruch 1, **dadurch gekennzeichnet, dass** die Magnete (52) seitlich neben den Enden der Wascharme angeordnet sind.

3. Reinigungsmaschine nach Anspruch 2, **dadurch gekennzeichnet, dass** die Magnete an Armen (54) gehaltert sind, die durch eine Endkappe (60) der Wascharme (3) festgelegt sind, die das freie Ende der Wascharme (3) verschließt.

4. Reinigungsmaschine nach einem der vorhergehenden Ansprüche, **dadurch gekennzeichnet, dass** die einzelnen Magnetdetektoren Reed-Schalter (53,54) sind.

5. Reinigungsmaschine nach Anspruch 4, **dadurch gekennzeichnet, dass** die einzelnen Reed-Schalter (53, 54) in den vertikal angeordneten Sensorrohren (51) vertikal übereinander angeordnet sind.

## Claims

1. Cleaning machine for cleaning medical, pharmaceutical, and/or laboratory utensils, comprising a washing chamber (40) adapted for the introduction of a trolley (10) for carrying objects to be washed, the utensils being placeable on individual tiered racks (11) thereof to which individual spraying arms (3) are assigned, which are adapted to rotate around a vertical rotational axis (B) and which are supplied with washing liquid through a rotary coupling, the spraying arms being provided with outlet openings (33), from which jets of washing liquid can be directed onto the objects located on the individual racks (11) of the trolley (10); and a rotation-monitoring system comprising magnetic detectors disposed on at least one of the side walls of the washing chamber (40), magnets (52) being arranged at the ends of the spraying arms (3) and adapted to actuate the magnetic detectors (53, 54) when the ends of the spraying arms (3) pass by them,
**characterized in that** the rotational axes (B) of the spraying arms of the trolley (10) introduced into the washing chamber (40) are offset from the centre plane (A) of the washing chamber (40) extending in the direction in which the trolley (10) is introduced; **in that** on both side walls of the washing chamber (40) sensor tubes (51) are arranged, within which a number of magnetic detectors (53, 54) assigned to a possible rotational plane of a spraying arm (3) are disposed, the detection range of the magnetic detectors (53, 54) encompassing the rotational plane of an individual spraying arm (3) and the area immediately above and immediately below it; and **in that** the magnets (52) mounted on the ends of the spraying arms (52) are adapted to actuate only the magnetic detectors which are closest resulting from the offset between the rotational axes (B) of the spraying arms (3) and the centre plane (A) of the spraying compartment (40).

2. Cleaning machine according to claim 1, **characterized in that** the magnets (52) are arranged laterally next to the ends of the spraying arms (3).

3. Cleaning machine according to claim 2, **characterized in that** the magnets (52) are mounted on arms (54) which are held in place on the spraying arms (3) by end caps (60) sealing off the free ends of the spraying arms (3).

4. Cleaning machine according to any of the preceding claims, **characterized in that** the individual magnetic detectors are reed switches (53, 54).

5. Cleaning machine according to claim 4, **characterized in that** the individual reed switches (53, 54) are arranged vertically one above the other in the vertical sensor tubes (51).

## Revendications

1. Machine à nettoyer pour le nettoyage d'ustensiles médicaux, pharmaceutiques et/ou de laboratoire, comprenant un espace de lavage (40) est un chariot de lavage de matériel à laver (10) qui peut être inséré dans l'espace de lavage (40), sachant que le chariot de lavage de matériel à laver présente plusieurs étages disposés les uns au-dessus des autres, sur lesquels les ustensiles de laboratoire peuvent être disposés, sachant que chaque étage a un bras de lavage (3), les bras de lavage (3) pouvant tourner sur un axe de rotation vertical (B) et pouvant être alimentés en liquide de lavage par un couplage rotatif, sachant que les bras de lavage sont dotés d'ouvertures de sortie (33) desquelles des jets de liquide de lavage peuvent être dirigés sur les objets se trouvant sur les différents étages (11) du chariot de lavage (10), sachant que la machine à nettoyer comprend en outre un système de surveillance de rotation, lequel présente des détecteurs magnétiques disposés sur au moins une paroi latérale de l'espace de lavage (40), et sachant que des aimants sont disposés sur les extrémités des bras de lavage, qui sont formés pour actionner les détecteurs magnétiques (53, 54) lorsque l'extrémité des bras de lavage (3) passe devant ceux-ci,
**caractérisé en ce que** les axes de rotation (B) des bras de lavage du chariot de lavage (10) inséré dans l'espace de lavage (40) sont décalés par rapport au plan médian (A) de l'espace de lavage (40) passant dans le sens d'insertion du chariot de lavage (10), **en ce que** sur les deux parois latérales de l'espace de lavage (40) sont placés des tubes de capteurs (51) dans lesquels un nombre des détecteurs magnétiques (53, 54) attribués à un étage de rotation possible du bras de lavage (3) sont disposés, dont l'aire de détection comprend le plan de rotation d'un bras de lavage respectif et l'aire placée immédiatement au-dessus et en-dessous, et **en ce que** les aimants (52) disposés sur les extrémités des bras de lavage (52) sont ainsi formés que seuls les détecteurs magnétiques les plus proches placés entre les axes de rotation (B) des bras de lavage (3) et le plan médian (A) de l'espace de lavage (40) du fait du décalage, soient actionnés.

2. Machine à nettoyer selon la revendication 1, **caractérisée en ce que** les aimants (52) sont disposés latéralement à côté des extrémités des bras de lavage.

3. Machine à nettoyer selon la revendication 2, **caractérisée en ce que** les aimants sont maintenus sur des bras (54), qui sont immobilisés par un bouchon d'extrémité (60) des bras de lavage (3) qui ferme l'extrémité libre des bras de lavage (3).

4. Machine à nettoyer selon l'une des revendications précédentes, **caractérisée en ce que** les détecteurs magnétiques individuels sont des interrupteurs reed (53, 54).

5. Machine à nettoyer selon la revendication 4, **caractérisée en ce que** les interrupteurs reed (53, 54) individuels sont disposés l'un sur l'autre verticalement dans les tubes de capteurs (51) disposés verticalement.
